# EUROPÄISCHE PATENTSCHRIFT

(11) **EP 2 428 517 B1**
(45) Veröffentlichungstag und Bekanntmachung des Hinweises auf die Patenterteilung: **06.11.2013**
(21) Anmeldenummer: 10176293.8
(22) Anmeldetag: 10.09.2010
(51) Int. Cl.: C07F 9/38

(54) **Synthese von tripodalen Bisphosphonatderivaten mit einem Adamantylgrundgerüst zur Funktionalisierung von Oberflächen**
Synthesis of tripodal bisphosphonate derivatives with an adamantyl base for functionalising surfaces
Synthèse de dérivés de bisphosphonates tripodaux dotés d'un équipement de base d'adamantyle pour la fonctionnalisation de surfaces

(43) Veröffentlichungstag der Anmeldung: 14.03.2012
(73) Patentinhaber: Justus-Liebig-Universität Gießen, 35390 Giessen (DE)
(72) Erfinder: Maison, Wolfgang, 35580, Wetzlar (DE); Khalil, Faiza, 35325, Mücke (DE); Franzmann, Elisa, 35435, Wettenberg-Wißmar (DE)
(74) Vertreter: Patentanwälte Olbricht Buchhold Keulertz

(56) Entgegenhaltungen:
- US-A1- 2006 063 834

## Beschreibung

Die vorliegende Erfindung beschreibt tripodale Bisphosphonatderivate mit einem Adamantylgrundgerüst zur Funktionalisierung von Oberflächen, Verfahren zu ihrer Herstellung sowie ihre Verwendung. Eine vierte verbliebene Position des Adamantangerüstes kann optional über sog. Click-Reaktionen beispielsweise mit Biomolekülen, Polyethylenglykol oder Wirkstoffen funktionalisiert werden.

### Beschreibung und Einleitung des allgemeinen Gebietes der Erfindung

Die vorliegende Erfindung betrifft die Gebiete organische Chemie und Materialwissenschaften.

### Stand der Technik

Der Stand der Technik kennt zahlreiche Methoden zur Funktionalisierung von Oberflächen. Solche Funktionalisierungen werden verwendet, um die Materialeigenschaften der Oberflächen gezielt zu verändern. Derartige Funktionalisierungen sollten möglichst beständig sein und eine hohe definierte Beladung der Oberfläche erlauben.

Im Bereich der Medizintechnik kommt funktionalisierten Oberflächen eine besondere Bedeutung zu. So sollten Implantate - beispielsweise im Dentalbereich und der Orthopädie (Gelenkersatz) möglichst biokompatibel sein, d.h. unter Anderem nicht zum Biofouling neigen, keine Entzündungsreaktionen hervorrufen und nicht von pathogenen Mikroorganismen besiedelt werden. Des Weiteren müssen sie starker mechanischer Beanspruchung dauerhaft standhalten.

Adamantan ist ein starres Molekül, das aus drei kondensierten, sechsgliedrigen carbocyclischen Ringen besteht. Die Kohlenstoffatome 1, 3, 5 und 7 des Adamantans sind Brückenkopfatome. Adamantanderivate sind bekannt und werden in Medizin und Materialwissenschaften verwendet. Wenn diese Adamantanderivate an drei Brückenkopfpositionen identische Substituenten tragen, weisen sie eine tripodale Anordnung auf.

Bisphosphonate gehören einer Medikamentengruppe an, die in den letzten 30 Jahren für diagnostische und therapeutische Zwecke bei Knochen- und Kalziumstoffwechselkrankheiten entwickelt wurde. Einige Verbindungen dieses Typs werden in Medikamenten zur Behandlung der Osteoporose verwendet. Sie sind in Deutschland zur Therapie der Osteoporose bei postmenopausalen Frauen, des Osteodystrophia deformans und der Tumor-assoziierten Hyperkalzämie zugelassen. Darüber hinaus finden sie Einsatz bei der Behandlung von Knochenmetastasen und fibröser Dysplasie.

Die US 2006/0063834 A1 beschreibt verschiedene Adamantanderivate mit tripodaler Anordnung, Verfahren zu ihrer Herstellung und ihre Verwendung für pharmazeutische Zusammensetzungen. Es werden jedoch keine Adamantanderivate offenbart, die zur Funktionalisierung von Oberflächen geeignet sind.

In A Oganesyan, lA Cruz, RB Amador, NA Sorto, J Lozano, CE Godinez, J Anguiano, H Pace, G Sabih, CG Gutierrez: "High Yield Selective Acylation of Polyamines: Proton as Protecting Group", Org Lett 2007, 9, 4967-4970 wird die selektive Acylierung von Polyaminen beschrieben, die mehrere identische oder ähnliche Aminfunktionen aufweisen. Die Autoren des Aufsatzes weisen darauf hin, dass die Allgegenwart von Polyamidbindungen in biologischen Molekülen die selektive Acylierung zu einem interessanten Ansatz für die Herstellung biomimetischer Moleküle macht. Allerdings werden keine Verbindungen offenbart, die substituierte 3,4-Dihydroxybenzylgruppen als Liganden des Adamantans aufweisen, welche zur Funktionalisierung von Oberflächen dienen.

Verfahren zur Herstellung starrer tripodaler Verbindungen auf Basis von Adamantan sind in W Maison, JV Frangioni, N Pannier: "Synthesis of Rigid Multivalent Scaffolds Based on Adamantane", Org Lett 2004, 6, 4567-4569 und in N Pannier, W Maison: "Rigid C3-Symmetric Scaffolds Based on Adamantane", Eur J Org Chem 2008, 1278-1284 sowie in K Nasr, N Pannier, JV Frangioni, W Maison: "Rigid Multivalent Scaffolds Based on Adamantane", J Org Chem 2008, 73, 1058-1060 beschrieben. Die Herstellung trivalenter Adamantangerüste mit Liganden umfassend Bisphosphonateinheiten ist dort nicht offenbart.

Aktuelle Forschungsarbeiten, die zum Ziel haben, knochenspezifische Therapeutika auf Basis von Bisphosphonaten zu finden, sind in S Zhang, G Gangal, H Uludag: "'Magic bullets' for bone diseases: progress in rational design of boneseeking medicinal agents", Chem Soc Rev 2007, 36, 507-531 beschrieben. In RS Ehrick, M Capaccio, DA Puleo, LG Bachas: "Ligand-Modified Aminobisphosphonate for Linking Proteins to Hydroxyapatite and Bone Surface", Bioconjugate Chem 2008, 19, 315-321, sind ein Syntheseweg zur Bindung von Tetraethyl(aminomethylen)bisphosphonat (AMB) an Biotin sowie die Bindung von AMB-Biotin an Hydroxylapatit beschrieben.

Bislang kennt der Stand der Technik noch keine Möglichkeiten, um Bisphosphonate und Adamantan dergestalt miteinander zu kombinieren, dass Bisphosphonateinheiten an trivalente Adamantangerüste gebunden werden.

Die vorliegende Erfindung stellt erstmals solche trivalenten Adamantangerüste mit Liganden umfassend Bisphosphonateinheiten bereit. Die erfindungsgemäßen Verbindungen bestehen aus tripodalen Grundgerüsten auf Basis des Adamantans, an die drei Bisphosphonateinheiten in Brückenkopfpositionen gebunden sind. Die verbleibende vierte Brückenkopfposition kann leicht über sog. Click-Reaktionen weiter funktionalisiert werden, beispielsweise mit Biomolekülen, Farbstoffen, Radiomarkern, Polyethylenglykol oder Wirkstoffen.

### Aufgabe

Aufgabe der Erfindung ist es, Verbindungen bereitzustellen, die eine beständige Funktionalisierung und eine hohe definierte Beladung von Oberflächen erlauben, sowie Verfahren zur Herstellung dieser Verbindungen.

### Lösung der Aufgabe

Die Aufgabe der Bereitstellung von Verbindungen, die eine beständige Funktionalisierung und eine hohe definierte Beladung von Oberflächen erlauben, wird erfindungsgemäß gelöst durch Verbindungen gemäß Formel (I): 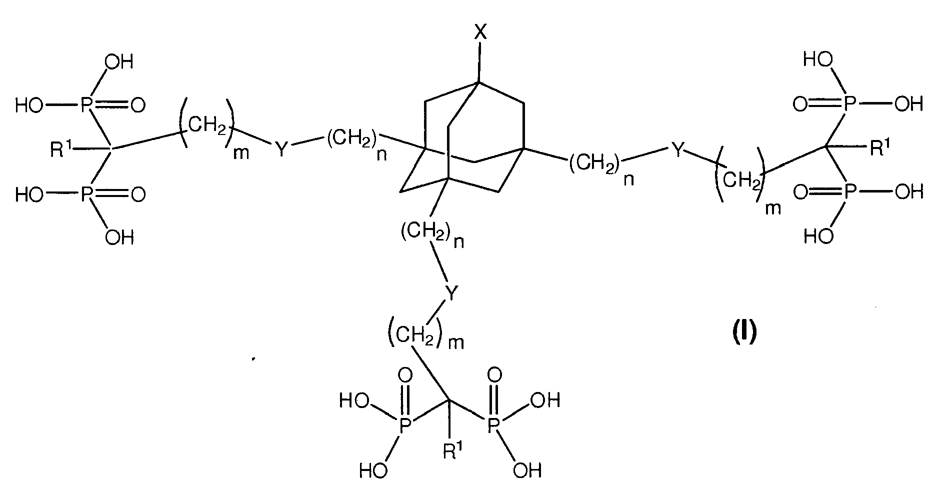 worin
- n und m unabhängig voneinander für eine ganze Zahl zwischen 0 und 10 stehen,
- R¹ ein Wasserstoffatom oder eine Hydroxygruppe ist,
- Y ausgewählt ist aus ―CH₂-, -CH=CH-, -CΞC-, -O-, -S-, -S-S-, -NH-, -O-NH-, -NH-O-, -HC=N-O-, -O-N=CH-, -NR²-, -Aryl-, -Heteroaryl-, -(C=O)-, -O-(C=O)-, -(C=O)-O-, -NH-(C=O)-, -(C=O)-NH-, -NR²-(C=O)-, -(C=O)-NR²-, -NH-(C=O)-NH-, -NH-(C=S)-NH-, wobei R² für eine lineare Alkylgruppe mit 1 bis 10 C-Atomen oder eine verzweigte oder cyclische Alkylgruppe mit 3 bis 10 C-Atomen steht,
   und
- X für eine Gruppe ―(CH₂)ₚ-R³ steht, worin p = 0-10 ist und R³ ausgewählt ist aus ―H, -NH₂, -NO₂, -OH, -SH, -O-NH₂, -NH-NH₂, -N=C=S-, -N=C=O-, -CH=CH₂, -CΞCH, -COOH, -(C=O)H, -(C=O)R⁴, wobei die Hydroxy-, Thio-, Amino- oder C=O-Gruppen optional durch eine Schutzgruppe geschützt sein können, -N₃, -OR⁴, -COOR⁴, -NHR⁴, - NR⁴R⁵, -CO-NHR⁴, -CONR⁴R⁵, -NH-CO-R⁴, 4-(2,5-Dioxopyrrol-1-yl), wobei R⁴ und R⁵ unabhängig voneinander für eine lineare Alkylgruppe mit 1 bis 10 C-Atomen, eine lineare Alkenyl- oder Alkinylgruppe mit 2 bis 10 C-Atomen, eine verzweigte Alkyl-, Alkenyl- oder Alkinylgruppe mit 3 bis 10 C-Atomen oder eine cyclische Alkyl- oder Alkenylgruppe mit 3 bis 10 C-Atomen stehen, oder
   X für eine verzweigte Alkyl-, Alkenyl- oder Alkinylgruppe mit 3 bis 10 C-Atomen oder eine cyclische Alkyl-, Alkenyl- oder Alkinylgruppe mit 3 bis 10 C-Atomen oder für eine Aryl- oder Heteroarylgruppe steht,
   wobei für den Fall, dass X eine verzweigte Alkyl-, Alkenyl- und Alkinylgruppe, eine cyclische Alkyl- oder Alkenylgruppe, eine Aryl- oder Heteroarylgruppe ist, optional ein C-Atom dieser Gruppe X eine Gruppe R³ gemäß obiger Definition tragen kann.

Die erfindungsgemäßen Verbindungen, das Verfahren zu ihrer Herstellung sowie die Verwendung dieser Verbindungen sind nachfolgend erläutert.

Die Erfindung ist nicht auf eine der nachfolgend beschriebenen Ausführungsformen beschränkt, sondern in vielfältiger Weise abwandelbar.

Sämtliche aus den Ansprüchen, der Beschreibung und den Zeichnungen hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Die erfindungsgemäßen Verbindungen gemäß Formel (1) erlauben eine beständige Funktionalisierung und eine hohe definierte Beladung von Oberflächen. Oberflächen, die funktionalisiert und beladen werden können, umfassen Metalle, Metalloxide, Apatit, Glas und Gemische davon. Der Begriff "Apatit" umfasst dabei sowohl Verbindungen der allgemeinen Formel Ca₅(PO₄)₃(F,Cl,OH), bei denen die Konzentration von Fluorid-, Chlorid- und Hydroxylionen frei austauschbar ist, als auch die Einzelminerale Fluorapatit, Chlorapatit und Hydroxylapatit.
Unter "hoher definierter Beladung" wird verstanden, dass die Beladung der Oberfläche eine lückenlose Beschichtung des Materials in Form einer Monolage ermöglicht. Unter "Monolage" wird dabei eine Schicht von erfindungsgemäßen Molekülen auf der Oberfläche verstanden, bei der die Schichthöhe nur ein Molekül beträgt. Eine "Funktionalisierung" ist die Hinzufügung funktioneller Gruppen auf die Oberfläche eines Materials durch chemische Synthesemethoden. Eine Beschichtung von Oberflächen mit den erfindungsgemäßen Verbindungen stellt daher eine Funktionalisierung dieser Oberflächen dar. An die Gruppe X kann optional ein Effektormolekül gebunden werden, was eine weitere Funktionalisierung darstellt. Ein Effektor ist ein Molekül oder ein Molekülbestandteil, welcher einen physikalischen, chemischen, biochemischen oder biologischen Prozess auslöst oder eine solche Wirkung kontrolliert, aktiviert oder inaktiviert. Beispielse für Effektoren sind Farbstoffe, radioaktive Moleküle, Biomoleküle wie Aminosäuren, Zucker, Peptide, Proteine, DNA, RNA, Polymere wie Ethylenglycol und Derivate davon sowie Wirkstoffe. Als Wirkstoffe werden Substanzen bezeichnet, die in geringer Dosis in einem Organismus eine spezifische Wirkung, eine Reaktion, hervorrufen.
Aufgrund der multivalenten Bindung der Verbindungen gemäß Formel (1) ist diese Funktionalisierung beständig gegenüber molekularen Austauschprozessen auf der Oberfläche (wie z.B. der Hydrolyse der Verknüpfung in wässrigen Medien) und auch gegenüber mechanischer Beanspruchung.

Dem Fachmann ist bekannt, dass cyclische Alkyl- und Alkenylgruppen mindestens drei Kohlenstoffatome enthalten müssen. Im Rahmen der vorliegenden Erfindung werden unter "ringförmigen" Gruppen solche Gruppen verstanden, bei denen alle Kohlenstoffatome an der Ringbildung beteiligt sind. "Cyclische" Gruppen können darüber hinaus noch acyclische Kohlenstoffatome enthalten. Ringförmige Alkyl- und Alkenylgruppen im Rahmen der vorliegenden Erfindung sind Propyl-, Butyl-, Pentyl-, Hexyl-, Heptyl-, Octyl-, Nonyl-, Decyl-, Propenyl-, Butenyl-, Pentenyl-, Hexenyl-, Heptenyl-, Octenyl-, Nonenyl- und Decenyl-Ringe. Handelt es sich bei den Gruppen X, R⁴ und/oder R⁵ um cyclische Alkyl- oder Alkenylgruppen, so werden diese ausgewählt aus den genannten ringförmigen Alkyl- und Alkenylgruppen, die keine weiteren Substituenten tragen, und aus den genannten ringförmigen Alkyl- und Alkenylgruppen, welche ihrerseits an eine oder mehrere acyclische Alkyl-, Alkenyl- oder Alkinylgruppen gebunden sind. Im letztgenannten Fall kann die Bindung der cyclischen Alkyl- oder Alkenylgruppe an das C1-Atom des Adamantangerüstes (falls die cyclische Gruppe X darstellt) bzw. an das entsprechende Atom der Gruppe R5 (falls die cyclische Gruppe R⁴ oder R⁵ darstellt) über ein cyclisches oder ein acyclisches Kohlenstoffatom der cyclischen Alkyl- oder Alkylengruppe erfolgen. Cyclische Alkylgruppen enthalten gemäß obiger Definition des Begriffes "Alkylgruppe" ebenfalls insgesamt maximal 10 Kohlenstoffatome.

Die Gruppe X ist erfindungsgemäß eine Gruppe ―(CH₂)ₚ-R³. Handelt es sich bei R³ um -NH₂, -OH, -SH, -O-NH₂, -NH-NH-COOH, -(C=O)H, -(C=O)R⁴, so können diese Gruppen optional durch eine Schutzgruppe geschützt sein. Schutzgruppen für Hydroxy-, Thiol-, Amino-, Carbonyl- und Carboxylgruppen sind dem Fachmann bekannt. Er kann diese Schutzgruppen verwenden, d.h. sie einführen und bei Bedarf wieder abspalten, ohne den Schutzbereich der Patentansprüche zu verlassen.

Beispielsweise, aber nicht erschöpfend, seien folgende Schutzgruppen genannt:
- für die OH-Gruppe: Methoxymethylether (MOM), β -Methoxyethoxymethylether (MEM), Silylether, 2-Tetrahydropyranyl (THP), Acetyl (Ac), Benzoyl (Bz), Benzyl (Bn, Bnl), Dimethoxytrityl (DMT), Methoxytrityl (MMT), p-Methoxybenzylether (PMB), Methylthiomethylether, Pivaloyl (Piv), Methylether, Ethoxyethylether (EE)
- für die SH-Gruppe: tert.-Butyl, 2-Tetrahydropyranyl, Acetyl, 2-Nitropyranyl, Phenacyl, (Cumarin-4-yl)methyl
- für die NH₂-Gruppe: 1-(1-Adamantyl)-1-methoxycarbonyl (ADPOC), Allyl-oxycarbonyl (ALLOC), Benzyloxycarbonyl (Z oder Cbz abgekürzt), 9-Fluorenylmethoxycarbonyl (FMOC), p-Methoxybenzylcarbonyl (Moz, Me-OZ), tert.-Butyloxycarbonyl (BOC), Acetyl (Ac), Benzoyl (Bz), Benzyl (Bn, Bnl), p-Methoxybenzyl (PMB), 3,4-Dimethoxybenzyl (DMPM), p-Methoxyphenyl (PMP), Tosyl (Ts), Sulfonamide
- für die Carbonylgruppe (in Aldehyden und Ketonen): die Umsetzung mit Diolen zu Acetalen bzw. Ketalen
- für die COOH-Gruppe: Methylester, Benzylester, tert.-Butylester, Silylester, Orthoester, Oxazoline

Unter Arylgruppen werden erfindungsgemäß Phenyl-, Naphthyl- und Anthracenylgruppen verstanden.
Heteroarylgruppe werden ausgewählt aus Furanyl, Pyrrolyl, Thiophenyl, Imidazolyl, Pyrazolyl, Triazolyl, Thiazolyl, Oxazolyl, Isooxazolyl, einem Oxadiazolyl, Pyridinyl, Pyrazinyl, Pyrimidinyl, Pyridazinyl, einem Triazinyl, einem Tetrazinyl, 1,4-Dioxinyl, einem Thiazinyl, einem Oxazinyl, einem Azepinyl, einem Diazepinyl, Benzofuranyl, Isobenzofuranyl, Indolyl, Isoindolyl, Benzothiophenyl, Ben-zo[c]thiophenyl, Benzimidazolyl, Purinyl, Indazolyl, Benzoxazolyl, Benzisoxazolyl, Benzothiazolyl, Chinolinyl, Isochinolinyl, Chinoxalinyl, Acridinyl, Chinazolinyl und Cinnolinyl.

In einer vorteilhaften Ausführungsform ist Y ausgewählt aus keinem Atom, -CH₂-, -NH-(C=O)-, -(C=O)-NH-, -NR²-, wobei R² wie oben definiert ist.

In einer weiteren vorteilhaften Ausführungsform ist n eine ganze Zahl zwischen 0 und 3.
In einer weiteren vorteilhaften Ausführungsform ist m eine ganze Zahl zwischen 0 und 3.
Besonders vorteilhaft stehen n und m unabhängig voneinander für ganze Zahlen zwischen 0 und 3.

In einer weiteren vorteilhaften Ausführungsform stellt X eine Gruppe (-CH₂)ₚ-R³ dar, wobei p eine ganze Zahl zwischen 0 und 3 darstellt und R³ wie in Anspruch 1 definiert ist.

In einer weiteren vorteilhaften Ausführungsform steht X für -(CH₂)ₚ-R³, worin R³ ausgewählt ist aus ―H, -OH, -NH₂, -NO₂, -NH-NH₂, -NHR⁴, -NR⁴R⁵, -O-NH₂, -NH-(C=O)-CΞCH, -CΞCH, -N=C=S, -N=C=O, -COOH, -(C=O)H, -(C=O)R⁴ und wobei p eine ganze Zahl zwischen 0 und 3 darstellt und R⁴ und R⁵ wie oben definiert sind.

Die erfindungsgemäßen Verbindungen gemäß Formel (1) werden hergestellt, indem eine Verbindung X-Ad[(CH₂)ₙ-Y']₃ mit einem Reagenz Y"C[PO(OH)_{2]2}R¹ zur korrespondierenden Verbindung X-Ad{(CH₂)ₙ-Y-C[PO(OH₂]₂R¹}3 umgesetzt und das Reaktionsprodukt anschließend gereinigt wird, worin Ad für das Adamantylgerüst und Y' für einen Precursor (Vorläufer) der Gruppe Y gemäß Formel (1) stehen und wobei X, R¹ und n wie in Formel (1) definiert sind.

Unter Precursor (Vorläufer) wird hierbei eine funktionelle Gruppe verstanden, die durch Reaktion mit einer weiteren als Precursor fungierenden funktionellen Gruppe oder einem weiteren als Precursor funktionierenden Reagenz in eine funktionelle Gruppe gemäß Formel (1) umgewandelt wird.

Verbindungen der Formel X-Ad[(CH₂)ₙ-Y']₃ sind bekannt. Der Fachmann kann sie entweder kommerziell erwerben oder mit Hilfe seines Fachwissen nach bekannten Synthesevorschriften selbst herstellen.

In einer vorteilhaften Ausführungsform ist X ein Wasserstoffatom.

In einer weiteren vorteilhaften Ausführungsform ist X eine Gruppe ―(CH₂)ₚ-R⁵, worin p eine ganze Zahl zwischen 0 und 3 darstellt und R⁵ ausgewählt ist aus -OH, -NH₂, -NH-NH₂, -NHR⁶, -NR⁶R⁷, -O-NH₂, -NH-(C=O)-CΞCH, -CΞCH, - N=C=S, -N=C=O, -COOH, -(C=O)H, -(C=O)R⁶, wobei R⁶ und R⁷ wie in Formel (1) definiert sind. Diese Gruppen können, wie bereits ausgeführt, optional durch eine Schutzgruppe (Pg) geschützt sein. Werden diese Gruppen geschützt, so geschieht dies vor der Umsetzung mit dem Reagenz Y"Z, so dass in diesem Fall Pg-X-Ad[(CH₂)ₙ-Y']₃ mit einem Reagenz Y"C[PO(OH)₂]₂R¹ zur korrespondierenden Verbindung Pg-X-Ad{(CH₂)ₙ-Y-C[PO(OH)₂]₂R¹}₃ umgesetzt wird.

Geeignete Schutzgruppen sind oben beschrieben. Dem Fachmann ist bekannt, wie man diese Schutzgruppen einführt und auch wieder entfernt. Er kann dieses Wissen anwenden, ohne den Schutzbereich der Patentansprüche zu verlassen.

Die Reinigung des Reaktionsproduktes erfolgt beispielsweise durch Entfernen des Lösungsmittels, Aufnehmen des Rückstandes in einer Mischung aus einem polar aprotischen Lösungsmittel wie Ethylacetat und einer verdünnten Mineralsäure, z.B. verdünnter Salzsäure, Waschen mit einer gesättigten KHSO₄-Lösung und Trockung.

In einer vorteilhaften Ausführungsform ist Y"C[PO(OH)₂]₂R¹ ein Amino- oder Alokohol- funktionalisiertes Bisphosphonat, wie beispielsweise Pamidronat oder Alendronat sowie deren geschützte Derivate. In diesem Fall wird X-Ad[(CH₂)ₙ-Y']₃ oder Pg-X-Ad[(CH₂)ₙ-Y']₃ mit Y"C[PO(OH)₂]₂R¹ in Gegenwart eines Aktivierungsreagenzes und ggf. eines Kupplungsadditivs umgesetzt. Dabei ist Y' ein Carbonsäurerest oder ein Derivat derselben. Geeignete Aktivierungsreagenzien sind beispielsweise EDC, DCC, DCl, PyClop, HBTU, HATU, HOSu, TBTU, T3P, BopCl und 3-Cl-1-Pyridiniumiodid. Als Kupplungsadditive eignen sich beispielsweise die dem Fachmann bekannten Substanzen HOBT, HOAt, HONB und NHS. Dem Fachmann ist bekannt, dass diese Reaktionen zweckmäßig unter Zugabe einer Base wie beispielsweise DIPEA durchgeführt werden. Dem Fachmann sind weiterhin verschiedene Lösungsmittel zur Verwendung in den genannten Verfahren bekannt. Er kann diese Kombinationen von Aktivierungsreagenzien, Kupplungsadditiven, Basen und Lösungsmitteln mit seinem üblichen Wissen und der Standardliteratur selbst herstellen.
Wurde eine Schutzgruppe Pg eingeführt und/oder geschützte Bisphosphonate verwendet, so werden diese Schutzgruppen zuletzt optional entfernt und das entschützte Produkt anschließend gereinigt.

In einer weiteren vorteilhaften Ausführungsform ist Y"C[PO(OH)₂]₂R¹ ein Carboxyl funktionalisiertes Bisphosphonat, wie beispielsweise Dicarboxypropan-1,1-diphosphonat (DPD) oder eines seiner geschützten Derivate. In diesem Fall wird X-Ad[(CH₂)ₙ-Y']₃ oder Pg-X-Ad[(CH₂)ₙ-Y']₃ mit Y"C[PO(OH)_{2]2}R¹ in Gegenwart eines Aktivierungsreagenzes und ggf. eines Kupplungsadditivs umgesetzt. Y' ist hierbei vorteilhaft eine Alkohol- oder Aminfunktion. Geeignete Aktivierungsreagenzien sind beispielsweise EDC, DCC, DCI, PyClop, HBTU, HATU, HOSu, TBTU, T3P, BopCl und 3-Cl-1-Pyridiniumiodid. Als Kupplungsadditive sind beispielsweise die dem Fachmann bekannten Substanzen HOBT, HOAt und HONB verwendbar. Dem Fachmann ist bekannt, dass diese Reaktionen zweckmäßig unter Zugabe einer Base wie beispielsweise DIPEA durchgeführt werden. Dem Fachmann sind weiterhin verschiedene Lösungsmittel zur Verwendung in den genannten Verfahren bekannt. Er kann diese Kombinationen von Aktivierungsreagenzien, Kupplungsadditiven, Basen und Lösungsmitteln mit seinem üblichen Wissen und der Standardliteratur selbst herstellen.

In einer weiteren vorteilhaften Ausführungsform ist Y"C[PO(OH)₂]₂R¹ ein Aminofunktionalisiertes Bisphosphonat, wie beispielsweise Pamidronat oder Alendronat sowie deren geschützte Derivate. X-Ad[(CH₂)ₙ-Y']₃ oder Pg-X-Ad[(CH₂)ₙ-Y']₃ werden mit Y"C[PO(OH)₂]₂R¹ in Gegenwart eines Reduktionsmittels umgesetzt. Y' ist dabei ein Aldehyd oder ein Keton. Geeignete Reduktionsmittel sind beispielsweise NaBH₄, NaBH₃CN, NaBH(OAC)₃ sowie H₂ und Metallkatalysatoren. Dem Fachmann sind verschiedene Lösungsmittel zur Verwendung in den genannten Verfahren bekannt. Er kann diese Kombinationen von Reduktionsmitteln und Lösungsmitteln mit seinem üblichen Wissen und der Standardliteratur selbst herstellen.

In einer weiteren vorteilhaften Ausführungsform ist Y"C[PO(OH)₂]₂R¹ ein Amino oder Alkohol funktionalisiertes Bisphosphonat, wie beispielsweise Pamidronat oder Alendronat sowie deren geschützte Derivate. X-Ad[(CH₂)ₙ-Y']₃ oder Prot-X-Ad[(CH₂)ₙ-Y']₃ mit einer geeigneten Austrittsgruppe Y' werden mit Y"C[PO(OH)₂]₂R¹ umgesetzt. Geeignete Austrittsgruppen sind beispielsweise - OTs, OMs, -OTf sowie Halogenide. Dem Fachmann sind verschiedene Lösungsmittel zur Verwendung in den genannten Verfahren bekannt. Er kann diese Kombinationen von Austrittsgruppen und Lösungsmitteln mit seinem üblichen Wissen und der Standardliteratur selbst herstellen.

In einer weiteren vorteilhaften Ausführungsform ist Y"C[PO(OH)₂]₂R¹ ein Aminofunktionalisiertes Bisphosphonat, wie beispielsweise Pamidronat oder Alendronat sowie deren geschützte Derivate. X-Ad[(CH₂)ₙ-Y']₃ oder Pg-X-Ad[(CH₂)ₙ-Y']₃ werden mit Y"C[PO(OH)₂]₂R¹ umgesetzt. Y' ist dabei ein Isothiocyanat oder ein Isocyanat. Dem Fachmann sind verschiedene Lösungsmittel zur Verwendung in den genannten Verfahren bekannt. Er kann diese Kombinationen von Reduktionsmitteln und Lösungsmitteln mit seinem üblichen Wissen und der Standardliteratur selbst herstellen.

In einer weiteren vorteilhaften Ausführungsform ist Y"C[PO(OH)₂]₂R¹ ein Carbonylfunktionalisiertes Bisphosphonat oder ein geschütztes Derivat davon. X-Ad[(CH₂)ₙ-Y']₃ oder Pg-X-Ad[(CH₂)ₙ-Y']₃ werden mit Y"C[PO(OH)₂]₂R¹ umgesetzt. Y' ist dabei ein O-Alkylhydroxylamin oder das korrespondierende Hydrohalogenid. Dem Fachmann sind verschiedene Lösungsmittel zur Verwendung in den genannten Verfahren bekannt.

In einer weiteren vorteilhaften Ausführungsform ist Y"C[PO(OH)₂]₂R¹ ein Azid funktionalisiertes Bisphosphonat oder ein geschütztes Derivat davon. X-Ad[(CH₂)ₙ-Y']₃ oder Pg-X-Ad[(CH₂)ₙ-Y']₃ werden in Gegenwart eines Kupferkatalysators mit Y"C[PO(OH)₂]₂R¹ umgesetzt. Y' ist dabei ein Alkin. Dem Fachmann sind verschiedene Lösungsmittel und Kupferkatalysatoren zur Verwendung in den genannten Verfahren bekannt. Er kann diese Kombinationen von Katalysatoren und Lösungsmitteln mit seinem üblichen Wissen und der Standardliteratur selbst herstellen.

In einer weiteren vorteilhaften Ausführungsform ist Y"C[PO(OH)₂]₂R¹ ein Alkin funktionalisiertes Bisphosphonat oder ein geschütztes Derivat davon. X-Ad[(CH₂)ₙ-Y']₃ oder Pg-X-Ad[(CH₂)ₙ-Y']₃ werden in Gegenwart eines Kupferkatalysators mit Y"C[PO(OH)₂]₂R¹ umgesetzt. Y' ist dabei ein Azid. Dem Fachmann sind verschiedene Lösungsmittel und Kupferkatalysatoren zur Verwendung in den genannten Verfahren bekannt. Er kann diese Kombinationen von Katalysatoren und Lösungsmitteln mit seinem üblichen Wissen und der Standardliteratur selbst herstellen.

Wurde eine Schutzgruppe Pg eingeführt, so wird diese zuletzt, d.h. nach der Bildung von Pg-X-Ad{(CH₂)ₙ-Y-C[PO(OH)₂]₂R¹}3, optional entfernt und das entschützte Produkt anschließend gereinigt.

Die erfindungsgemäßen Verbindungen gemäß Formel (1) können in einem Verfahren zur Funktionalisierung von Oberflächen verwendet werden. Die Funktionalisierung geschieht dabei mittels dip and rinse, indem die zu funktionalisierenden Oberflächen in eine Lösung der erfindungsgemäßen Verbindungen getaucht werden.

Vorteilhaft werden die erfindungsgemäßen Verbindungen in einer wässrigen Pufferlösung gelöst, die eine Salzkonzentration aufweist, welche signifikant höher als physiologische Salzkonzentration (0,9 Gew.-% NaCl) ist. Ein geeigneter Puffer ist beispielsweise MOPS (3(N—Morpholin)-propansulfonsäure), geeignete Salze sind beispielsweise NaCl und K₂SO₄ und Gemische davon. Die Salzkonzentration beträgt vorteilhaft zwischen 10 und 20 Gew.-% und die Pufferkonzentration 0,05 bis 0,2 mmol.

Optional kann die Gruppe X der erfindungsgemäßen Verbindungen an einen Effektor gekuppelt sein. Die Kupplung von X an den Effektor kann dabei sowohl in Lösung, d.h. vor der Oberflächenfunktionalisierung, als auch auf der Oberfläche, d.h. nach der Oberflächenfunktionalisierung, erfolgen. Effektoren sind beispielsweise Ethergruppierungen, Estergruppierungen, Heteroaromaten, Farbstoffe, Metallkomplexe, Polymere (beispielsweise Polyethylenglykole), pharmazeutische Wirkstoffe (beispielsweise Antibiotika, Bisphosphonate), Biomoleküle (z.B. eine Aminosäure), Peptide, Kohlenhydrate und Terpene. Ist der Effektor ein Polymer und handelt es sich dabei um ein Polyethylenglykol, so handelt es sich vorteilhaft um eine Gruppierung ―(O-CH₂-CH₂)_{q}-R³ oder ―(CH₂-CH₂-O)_{q}-R³, worin q eine Zahl zwischen 1 und 10 und R³ wie unter Formel 1 beschrieben definiert ist.

In einer vorteilhaften Ausführungsform erfolgt die Kupplung von X dabei mittels Click-Chemie. Unter "Click-Reaktionen" versteht der Fachmann energetisch begünstigte Reaktionen, die spezifisch verlaufen und ein einziges Produkt ergeben. Es handelt sich damit um sehr einfach durchzuführende und effiziente Reaktionen. Click-Reaktionen finden Anwendung in Molekularbiologie, Wirkstoffentwicklung, Biotechnologie, makromolekularer Chemie und Materialwissenschaften.
Das Konzept der Click-Reaktion wurde von K. Barry Sharpless begründet und beschreibt Reaktionen, die
- modular aufgebaut sind,
- ein breites Anwendungsspektrum aufweisen,
- mit hohen Ausbeuten durchführbar sind,
- stereospezifisch ablaufen,
- einfache Reaktionsbedingungen erlauben (möglichst unempfindlich gegen Wasser und Sauerstoff),
- in umweltfreundlichen und/oder leicht entfernbaren Lösungsmitteln wie Wasser oder lösungsmittelfrei ablaufen,
- eine einfache Aufreinigung (Extraktion, Phasentrennung, Destillation oder Kristallisation) oder gar keine Aufreinigung benötigen.

"Click-Reaktionen sind in der Regel stark thermodynamisch begünstigt, häufig mehr als 84 kJ/mol, was in einem schnellen Umsatz mit hoher Selektivität für ein einzelnes Produkt resultiert. Dabei handelt es sich meist um Kohlenstoff-Heteroatom-Bindungsbildungen.

Chemische Reaktionen, die diese Kriterien erfüllen, sind:
- die Carbonylchemie des "Non-Aldol-Typs" wie z.B. die Bildung von Harnstoff, Thioharnstoff, Oximen, Iminen, aromatischen Heterocyclen und Hydrazonen sowie die Bildung von Carbamiden und Amiden,
- Cycloadditionen an ungesättigten C-C-Bindungen, besonders 1,3-dipolare Cycloadditionen wie die Huisgen-Cycloaddition, außerdem Diels-Alder-Reaktionen,
- nucleophile Substitutionen, besonders die Ringöffnung von gespannten, elektrophilen Heterozyklen wie Aziridinen und Epoxiden,
- Additionsreaktionen an C-C-Mehrfachbindungen, meist oxidativ wie z.B. Epoxidierung, Aziridierung oder Dihydroxylierung, aber auch Michaeladditionen von Nu-H, wobei Nu ein Nucleophil ist.

In einer weiteren vorteilhaften Ausführungsform erfolgt die Kupplung des Effektors an die Gruppe X über konventionelle Substitutions- oder Additionsreaktionen, die nicht die oben genannten Bedingungen einer Click-Reaktion gehören. Zu diesen konventionellen Reaktionen zählen beispielsweise die Etherbildung, die Veresterung einer Carbonsäure oder die Amidbildung.

Besonders vorteilhaft handelt es sich bei den Oberflächen, die funktionalisiert werden sollen, um metallische Oberflächen umfassend Eisen und/oder Titan oder um Oberflächen umfassend Apatit und/oder Glas. Dem Fachmann ist bekannt, dass Knochen von Wirbeltieren zu etwa 50 % aus Apatit bestehen, Dentin zu etwa 70 % und Zahnschmelz zu mehr als 95 %. Moderner Zahnersatz, beispielsweise Füllungen und Zahnimplantate, umfasst häufig Apatit und/oder Vorrichtungen, die Eisen und/oder Titan umfassen. Es ist ferner bekannt, dass die Oberflächen von Endoprothesen, z.B. für Hüft- und Kniegelenke, Eisen und/oder Titan umfassen. Die erfindungsgemäßen Verbindungen gemäß Formel (l) sowie die daraus erhältlichen, an einen Effektor gekuppelten Verbindungen eignen sich daher zur Oberflächenfunktionalisierung von Zahnersatz und Gelenksendoprothesen.

### Ausführungsbeispiel 1:

### Herstellung von 3,5,7-tris[2-(N-Hydroxysuccinimido)-carboxyethyl]-adamantan 1

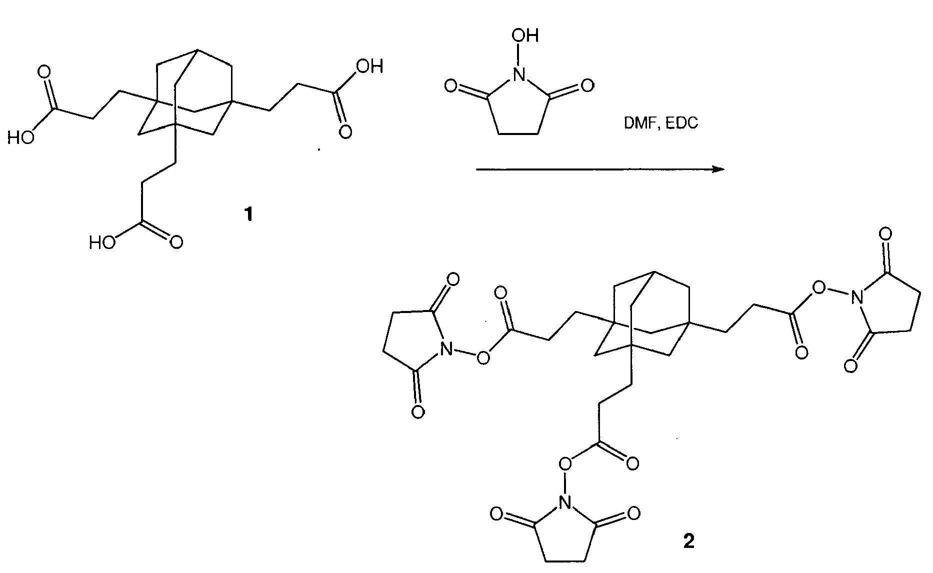

Zu einer Lösung von 500 mg (1.42 mmol) der Tricarbonsäure **1** und 588 mg (5.11 mmol) N-Hydroxysucccinimid in 50 ml absolutiertem DMF wurden 979 mg (5.11 mmol) EDC-Hydrochlorid (1-Ethyl-3(3-dimethylaminopropyl)carbodiimid) bei Raumtemperatur gegeben. Die Reaktionslösung wurde für 24 h bei Raumtemperatur gerührt und anschließend das Lösungsmittel abdestilliert. Der erhaltene Rückstand wurde in Ethylacetat aufgenommen, drei mal mit Wasser gewaschen, über Na₂SO₄ getrocknet und das Lösungsmittel abdestilliert. Es konnten 447 mg eines farblosen Feststoffes **2** als Rohprodukt erhalten werden, welcher ohne weitere Reinigung verwendet wurde.
**¹H-NMR (400 MHz, CDCl₃):** δ [ppm] = 2.80 (m, 12 H, 9-H), 2.57 (t, 6H, ³*J* = 9.0 Hz, 6-H), 1.60 (t, 6H, ³*J* = 8.0 Hz, 5-H), 1.37 (m, 6H, 2-H), 1.20 (d, 3 H, ²*J* = 12.0 Hz, 4a-H), 1.14 (d, 3H, ²*J* = 12.0 Hz, 4b-H); **¹³C-NMR (100 MHz, CDCl₃):** δ [ppm] = 169.5 (C7), 169.4 (C8), 40.6 (C2), 37.4 (C5), 33.5 (C3), 31.6 (C6), 29.1 (C1), 25.6 (C9)
**MS-ESI m/z (%)**: 666.3 [MNa]⁺(100), 1309.9 [2xMNa]+(11)

### Ausführungsbeispiel 2:

### Herstellung von Verbindung 3

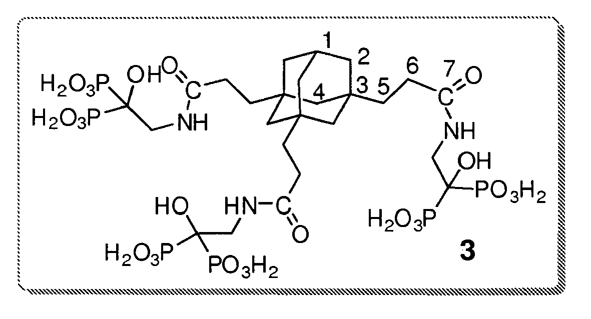

100 mg (0.155 mmol) der NHS-Ester Verbindunge **2** wurden in 10 mL DCM, 10 mL DMF und 40 H₂O gelöst und mit 4.3 mL (31.07 mmol) dest. Et₃N und 369 mg (1.55 mmol) Pamidronat versetzt und 5 Tage bei Raumtemperatur gerührt.
Das Lösungsmittel wurde bis zur Trockenheit eingeengt und das Rohprodukt wurde mittels RP-HPLC (RP8) säulenchromatographisch gereinigt. (Vorab MS Messungen, ergaben einen vollständigen Umsatz) Es wurde ein Gradient von 1%Acetonitril, 99% Wasser isokratisch 15 min gefahren.
**MS-ESI m/z** (%): 960.1 [M-H]⁻(100)

## Patentansprüche

1. Verbindungen gemäß Formel (I): 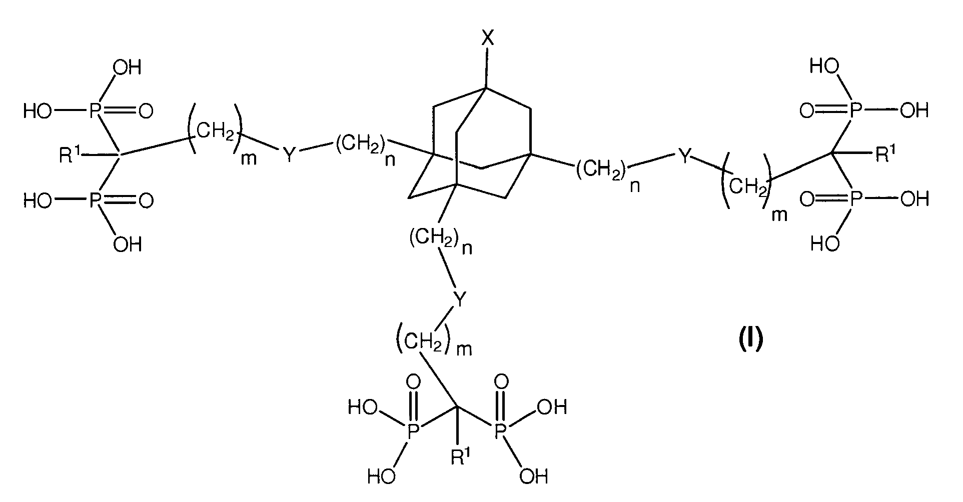 worin
- n und m unabhängig voneinander für eine ganze Zahl zwischen 0 und 10 stehen,
- R¹ ein Wasserstoffatom oder eine Hydroxygruppe ist,
- Y ausgewählt ist aus -CH₂-, -CH=CH-, -C≡C-, -O-, -S-, -S-S-, -NH-, -O-NH-, -NH-O-, -HC=N-O-, -O-N=CH-, -NR²-, -Aryl-, -Heteroaryl-, -(C=O)-, -O-(C=O)-, -(C=O)-O-, -NH-(C=O)-, -(C=O)-NH-, -NR²-(C=O)-, -(C=O)-NR²-, -NH-(C=O)-NH-, -NH-(C=S)-NH-, wobei R² für eine lineare Alkylgruppe mit 1 bis 10 C-Atomen oder eine verzweigte oder cyclische Alkylgruppe mit 3 bis 10 C-Atomen steht,
und
- X für eine Gruppe -(CH₂)ₚ-R³ steht, worin p = 0-10 ist und R³ ausgewählt ist aus -H, -NH₂, -NO₂, -OH, -SH, -O-NH₂, -NH-NH₂, -N=C=S-, -N=C=O-, -CH=CH₂, -C=CH, -COOH, -(C=O)H, -(C=O)R⁴, wobei die Hydroxy-, Thio-, Amino- oder C=O-Gruppen optional durch eine Schutzgruppe geschützt sein können, -N₃, -OR⁴, -COOR⁴, -NHR⁴,-NR⁴R⁵, -CO-NHR⁴, -CONR⁴R⁵, -NH-CO-R⁴, 4-(2,5-Dioxopyrrol-1-yl), wobei R⁴ und R⁵ unabhängig voneinander für eine lineare Alkylgruppe mit 1 bis 10 C-Atomen, eine lineare Alkenyl- oder Alkinylgruppe mit 2 bis 10 C-Atomen, eine verzweigte Alkyl-, Alkenyl- oder Alkinylgruppe mit 3 bis 10 C-Atomen oder eine cyclische Alkyl- oder Alkenylgruppe mit 3 bis 10 C-Atomen stehen, oder
X für eine verzweigte Alkyl-, Alkenyl- oder Alkinylgruppe mit 3 bis 10 C-Atomen oder eine cyclische Alkyl-, Alkenyl- oder Alkinylgruppe mit 3 bis 10 C-Atomen oder für eine Aryl- oder Heteroarylgruppe steht,
wobei für den Fall, dass X eine verzweigte Alkyl-, Alkenyl- und Alkinylgruppe, eine cyclische Alkyl- oder Alkenylgruppe, eine Aryl- oder Heteroarylgruppe ist, optional ein C-Atom dieser Gruppe X eine Gruppe R³ gemäß obiger Definition tragen kann.

2. Verbindung gemäß Anspruch 1, **dadurch gekennzeichnet, dass** Y ausgewählt ist aus keinem Atom, -CH₂-,-NH-(C=O)-, -(C=O)-NH-, -NR¹-, wobei R² wie in Anspruch 1 definiert ist.

3. Verbindung gemäß einem der Ansprüche 1 und 2, **dadurch gekennzeichnet, dass** n eine ganze Zahl zwischen 0 und 3 ist.

4. Verbindung gemäß einem der Ansprüche 1 bis 3, **dadurch gekennzeichnet, dass** m eine ganze Zahl zwischen 0 und 3 ist.

5. Verbindung gemäß einem der Ansprüche 1 bis 4, **dadurch gekennzeichnet, dass** X eine Gruppe (-CH₂)ₚ-R³ darstellt, wobei p eine ganze Zahl zwischen 0 und 3 darstellt und R³ wie in Anspruch 1 definiert ist.

6. Verbindung gemäß einem der Ansprüche 1 bis 5, **dadurch gekennzeichnet, dass** X eine Gruppe -(CH₂)ₚ-R³ darstellt, worin R³ ausgewählt ist aus -H, -OH, -NH₂, -NO₂, -NH-NH₂, -NHR⁴, -NR⁴R⁵, -O-NH₂, -NH-(C=O)-C≡CH, -C≡CH, -N=C=S, -N=C=O, -COOH, -(C=O)H, -(C=O)R⁴ und wobei p eine ganze Zahl zwischen 0 und 3 darstellt und R⁴ und R⁵ wie in Anspruch 1 definiert sind.

7. Verfahren zur Herstellung von Verbindungen gemäß einem der Ansprüche 1 bis 6, **dadurch gekennzeichnet, dass** eine Verbindung X-Ad[(CH₂)ₙ-Y']₃ mit einem Reagenz Y"C[PO(OH)₂]₂R¹ zur korrespondierenden Verbindung X-Ad{(CH₂)ₙ-Y-C[PO(OH)₂]₂R¹}₃ umgesetzt und das Reaktionsprodukt anschließend gereinigt wird, wobei Ad für das Adamantylgerüst und Y' für einen Precursor (Vorläufer) der Gruppe Y gemäß Formel (I) stehen und worin X, R¹ und n wie in Anspruch 1 definiert sind.

8. Verfahren gemäß Anspruch 7, **dadurch gekennzeichnet, dass** X ein Wasserstoffatom ist.

9. Verfahren gemäß Anspruch 7, **dadurch gekennzeichnet, dass** X eine Gruppe -(CH₂)ₚ-R³ ist, worin p eine ganze Zahl zwischen 0 und 3 darstellt und R³ ausgewählt ist aus -OH, -NH₂, -NO₂, -NH-NH₂, -NHR⁴, -NR⁴R⁵, -O-NH₂, -NH-(C=O)-C≡CH, -C≡CH, -N=C=S, -N=C=O, -COOH, -(C=O)H, -(C=O)R⁴, wobei R⁴ und R⁵ wie in Formel (I) definiert sind.

10. Verfahren gemäß Anspruch 9, **dadurch gekennzeichnet, dass** die Gruppe R³ vor der Umsetzung mit dem Reagenz Y"C[PO(OH)₂]₂R¹ durch eine Schutzgruppe Pg geschützt wird, so dass die Verbindung Pg-X-Ad[(CH₂)ₙ-Y']₃ mit einem Reagenz Y"C[PO(OH)₂]₂R¹ zur korrespondierenden Verbindung Pg-X-Ad{(CH₂)ₙ-Y-C[PO(OH)₂]₂R¹}₃ umgesetzt wird.

11. Verwendung von Verbindungen gemäß einem der Ansprüche 1 bis 6 in einem Verfahren zur Funktionalisierung von Oberflächen, wobei die Funktionalisierung mittels dip and rinse erfolgt.

12. Verwendung von Verbindungen gemäß Anspruch 11, **dadurch gekennzeichnet, dass** die funktionelle Gruppe X der Verbindungen gemäß Formel (I) zuerst mit einem Effektor gekoppelt wird und danach die Funktionalisierung der Oberfläche mittels dip and rinse erfolgt.

13. Verwendung von Verbindungen gemäß Anspruch 11, **dadurch gekennzeichnet, dass** zuerst die Funktionalisierung der Oberflächen mittels dip and rinse erfolgt und danach die funktionelle Gruppe X der Verbindungen gemäß Formel (I) mit einem Effektor gekoppelt wird.

## Claims

1. Compounds according to formula (I): 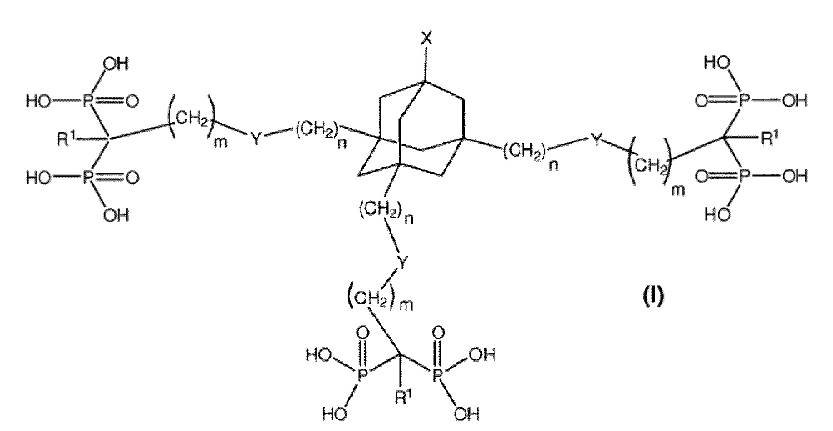 wherein
- n and m independently represent an integer between 0 and 10,
- R¹ is a hydrogen atom or a hydroxyl group,
- Y is selected from -CH₂-, -CH=CH-, -C≡C-, -O-, -S-, -S-S-, -NH-, -O-NH-, -NH-O-, -HC=N-O-, -O-N=CH-, -NR²-, -aryl-, -heteroaryl-, -(C=O)-, -O-(C=O)-, -(C=O)-O-, -NH-(C=O)-, -(C=O)-NH-, -NR²-(C=O)-, -(C=O)-NR²-, -NH-(C=O)-NH-, -NH-(C=S)-NH-, wherein R² represents a linear alkyl group having 1 to 10 C atoms or a branched or cyclic alkyl group having 3 to 10 C atoms,
and
- X represents a group -(CH₂)ₚ-R³, wherein p = 0-10 and R³ is selected from -H, -NH₂, -NO₂, -OH, -SH, -O-NH₂, -NH-NH₂, -N=C=S-, -N=C=O-, -CH=CH₂, -C≡CH, -COOH, -(C=O)H, -(C=O)R⁴ wherein the hydroxyl, thio, amino or C=O groups optionally can be protected by a protecting group, -N₃, -OR⁴, -COOR⁴, -NHR⁴, -NR⁴R⁵, -CO-NHR⁴, -CONR⁴R⁵, -NH-CO-R⁴, 4-(2,5-dioxopyrrol-1-yl), wherein R⁴ and R⁵ independently represent a linear alkyl group having 1 to 10 C atoms, a linear alkenyl or alkynyl group having 2 to 10 C atoms, a branched alkyl, alkenyl or alkynyl group having 3 to 10 C atoms or a cyclic alkyl or alkenyl group having 3 to 10 C atoms, or
- X represents a branched alkyl, alkenyl or alkynyl group having 3 to 10 C atoms or a cyclic alkyl, alkenyl or alkynyl group having 3 to 10 C atoms or an aryl or heteroaryl group,
wherein, if X is a branched alkyl, alkenyl and alkynyl group, a cyclic alkyl or alkenyl group, an aryl or heteroaryl group, a C atom of this group X optionally can carry a group R³ according to the definition above.

2. Compound according to claim 1, **characterised in that** Y is selected from no atom, -CH₂-, -NH-(C=O)-, -(C=O)-NH-, -NR¹-, R² being defined as in claim 1.

3. Compound according to either claim 1 or claim 2, **characterised in that** n is an integer between 0 and 3.

4. Compound according to any of claims 1 to 3, **characterised in that** m is an integer between 0 and 3.

5. Compound according to any of claims 1 to 4, **characterised in that** X represents a group (-CH₂)ₚ-R³, p representing an integer between 0 and 3 and R³ being defined as in claim 1.

6. Compound according to any of claims 1 to 5, **characterised in that** X represents a group -(CH₂)ₚ-R³, R³ being selected from -H, -OH, -NH₂, -NO₂, -NH-NH₂, -NHR⁴, -NR⁴R⁵, -O-NH₂, -NH-(C=O)-C≡CH, -C=CH, -N=C=S, -N=C=O, -COOH, -(C=O)H, -(C=O)R⁴ and p representing an integer between 0 and 3 and R⁴ and R⁵ being defined as in claim 1.

7. Method for producing compounds according to any of claims 1 to 6, **characterised in that** a compound X-Ad[(CH₂)ₙ-Y']₃ is converted by means of a reagent Y"C[PO(OH)₂]₂R' to the corresponding compound X-Ad{(CH₂)ₙ-Y-C[PO(OH)₂]₂R¹}₃ and the reaction product is subsequently purified, Ad representing the adamantyl skeleton and Y' representing a precursor of the group Y according to formula (I) and X, R¹ and n being defined as in claim 1.

8. Method according to claim 7, **characterised in that** X is a hydrogen atom.

9. Method according to claim 7, **characterised in that** X is a group -(CH₂)ₚ-R³, p representing an integer between 0 and 3 and R³ being selected from -OH, -NH₂, -NO₂, -NH-NH₂, -NHR⁴, -NR⁴R⁵, -O-NH₂, -NH-(C=O)-C≡CH, -C≡CH, -N=C=S, -N=C=O, -COOH, -(C=O)H, -(C=O)R⁴, R⁴ and R⁵ being defined as in formula (I).

10. Method according to claim 9, **characterised in that** the group R³ is protected against the conversion by means of the reagent Y"C[PO(OH)₂]₂R¹ by a protecting group Pg, such that the compound Pg-X-Ad[(CH₂)ₙ-Y']₃ is converted by means of a reagent Y"C[PO(OH)₂]₂R¹ to the corresponding compound Pg-X-Ad{(CH₂)ₙ-Y-C[PO(OH)₂]₂R¹}₃.

11. Use of compounds according to any of claims 1 to 6 in a method for the functionalization of surfaces, wherein the functionalization is carried out by means of dip and rinse.

12. Use of compounds according to claim 11, **characterised in that** the functional group X of the compounds according to formula (I) are firstly coupled to an effector, and then the functionalization of the surface is carried out by means of dip and rinse.

13. Use of compounds according to claim 11, **characterised in that** firstly, the functionalization of the surfaces is carried out by means of dip and rinse, and then the functional group X of the compounds according to formula (I) is coupled to an effector.

## Revendications

1. Composés selon la formule (I) : 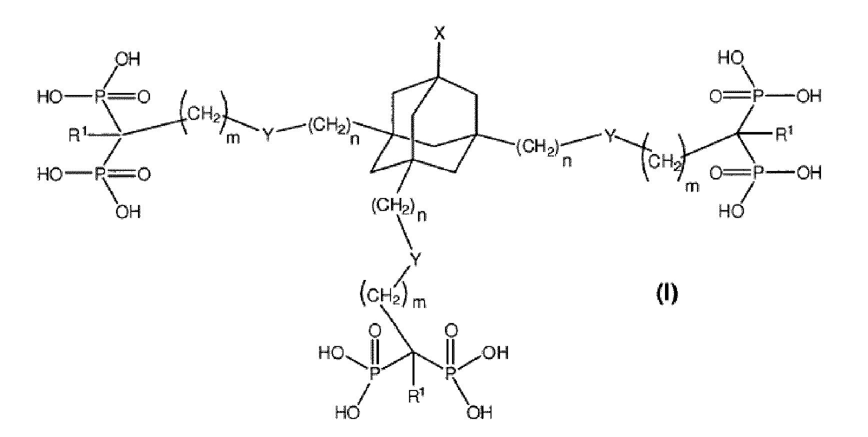 dans laquelle
- n et m sont indépendamment l'un de l'autre, un nombre entier entre 0 et 10,
- R¹ est un atome d'hydrogène ou un groupe hydroxy,
- Y est choisi parmi -CH₂-, -CH=CH-, -C≡C-, -O-, -S-, -S-S-, -NH-, -O-NH-, -NH-O-, -HC=N-O-, -O-N=CH-, -NR²-,-aryle-, -hétéroaryle-, -(C=O)-, -O-(C=O)-, -(C=O)-O-, - NH-(C=O)-, -(C=O)-NH, -NR²-(C=O)-, -(C=O)-NR²-, -NH-(C=O)-NH-, -NH-(C=S)-NH-, où R² est un groupe alkyle linéaire comportant 1 à 10 atomes de C ou un groupe alkyle ramifié ou cyclique comportant 3 à 10 atomes de C
et
- X est un groupe -(CH₂)ₚ-R³, où p = 0 à 10
et R³ est choisi parmi -H, -NH₂, -NO₂, -OH, -SH, -O-NH₂, -NH-NH₂, -N=C=S-, -N=C=O-, -CH=CH₂, -C≡CH, -COOH, -(C=O)H, - (C=O)R⁴, où les groupes hydroxy, thio, amino ou C=O peuvent être éventuellement protégés par un groupe protecteur, -N₃, -OR⁴, -COOR⁴, -NHR⁴, -NR⁴R⁵, -CO-NHR⁴, - CONR⁴R⁵, -NH-CO-R⁴, 4-(2,5-dioxopyrrol-1-yl), où R⁴ et R⁵ sont indépendamment l'un de l'autre, un groupe alkyle linéaire comportant 1 à 10 atomes de C, un groupe alcényle ou alcinyle linéaire comportant 2 à 10 atomes de C, un groupe alkyle, alcényle ou alcinyle ramifié comportant 3 à 10 atomes de C ou un groupe alkyle ou alcényle cyclique comportant 3 à 10 atomes de C, ou
X est un groupe alkyle, alcényle ou alcinyle ramifié comportant 3 à 10 atomes de C ou un groupe alkyle, alcényle ou alcinyle cyclique comportant 3 à 10 atomes de C ou un groupe aryle ou hétéroaryle,
où, dans le cas où X est un groupe alkyle, alcényle ou alcinyle ramifié, un groupe alkyle ou alcényle cyclique, un groupe aryle ou hétéroaryle, éventuellement un atome de C de ce groupe X peut porter un groupe R³ selon la définition ci-dessus.

2. Composé selon la revendication 1, **caractérisé en ce que** Y est choisi parmi aucun atome, -CH₂-, -NH-(C=O), - (C=O)-NH-, -NR¹-, où R² est tel que défini dans la revendication 1.

3. Composé selon l'une des revendications 1 et 2, **caractérisé en ce que** n est un nombre entier entre 0 et 3.

4. Composé selon l'une des revendications 1 à 3, **caractérisé en ce que** m est un nombre entier entre 0 et 3.

5. Composé selon l'une des revendications 1 à 4, **caractérisé en ce que** X est un groupe (-CH₂)ₚ-R³, où p est un nombre entier entre 0 et 3 et R³ est tel que défini dans la revendication 1.

6. Composé selon l'une des revendications 1 à 5, **caractérisé en ce que** X est un groupe - (CH₂)ₚ-R³, où R³ est choisi parmi -H, -OH, -NH₂, -NO₂, -NH-NH₂, -NHR⁴, - NR⁴R⁵, -O-NH₂, -NH-(C=O)-C≡CH, -C≡CH, -N=C=S, -N=C=O, - COOH, -(C=O)H, -(C=O)R⁴, et où p est un nombre entier entre 0 et 3 et R⁴ et R⁵ sont tels que définis dans la revendication 1.

7. Procédé de production de composés selon l'une des revendications 1 à 6, **caractérisé en ce qu'**on fait réagir un composé X-Ad[(CH₂)ₙ-Y']₃ avec un réactif Y"C[PO(OH)₂]₂R¹ en composé correspondant X-Ad{(CH₂)ₙ-Y-C[PO(OH)₂]₂R¹}₃ et le produit réactionnel est ensuite purifié, où Ad est un squelette adamantyle et Y' est un précurseur du groupe Y selon la formule (I) et où X, R¹ et n sont tels que définis dans la revendication 1.

8. Procédé selon la revendication 7, **caractérisé en ce que** X est un atome d'hydrogène.

9. Procédé selon la revendication 7, **caractérisé en ce que** X est un groupe -(CH₂)ₚ-R³, où p est un nombre entier entre 0 et 3 et R³ est choisi parmi -OH, -NH₂, -NO₂, - NH-NH₂, -NHR⁴, -NR⁴R⁵, -O-NH₂, -NH-(C=O)-C≡CH, -C≡CH, - N=C=S, -N=C=O, -COOH, -(C=O)H, -(C=O)R⁴, où R⁴ et R⁵ sont tels que définis dans la formule (I).

10. Procédé selon la revendication 9, **caractérisé en ce que** le groupe R³ est protégé avant la réaction avec le réactif Y"C[PO(OH)₂]₂R¹ par un groupe protecteur Pg de sorte que la liaison Pg-X-Ad[(CH₂)ₙ-Y']₃ réagisse avec un réactif Y"C[PO(OH)₂]₂R¹ pour donner un composé correspondant Pg-X-Ad{(CH₂)ₙ-Y-C [PO (OH)₂]₂R¹}₃.

11. Utilisation de composés selon l'une des revendications 1 à 6, dans un procédé de fonctionnalisation de surfaces, la fonctionnalisation s'effectuant par trempage et rinçage.

12. Utilisation de composés selon la revendication 11, **caractérisé en ce que** le groupe fonctionnel X des composés selon la formule (I) est d'abord couplé à un effecteur et ensuite s'effectue la fonctionnalisation de la surface par trempage et rinçage.

13. Utilisation de composés selon la revendication 11, **caractérisée en ce que** la fonctionnalisation des surfaces s'effectue tout d'abord par trempage et rinçage et ensuite, le groupe fonctionnel X des composés selon la formule (I) est couplé à un effecteur.
